(19) Europäisches Patentamt / European Patent Office / Office européen des brevets

(11) **EP 2 571 423 B1**

(12) **EUROPEAN PATENT SPECIFICATION**

(45) Date of publication and mention
of the grant of the patent:
**21.03.2018 Bulletin 2018/12**

(21) Application number: **11722912.0**

(22) Date of filing: **27.04.2011**

(51) Int Cl.:
**A61B 5/087** (2006.01)

(86) International application number:
**PCT/IB2011/051848**

(87) International publication number:
**WO 2011/145014 (24.11.2011 Gazette 2011/47)**

(54) **SYSTEM FOR ESTIMATING UPPER AIRWAY RESISTANCE AND LUNG COMPLIANCE EMPLOYING INDUCED CENTRAL APNEAS**

SYSTEM ZUR BEURTEILUNG DER RESISTENZ DER OBEREN ATEMWEGE UND LUNGENCOMPLIANCE MIT INDUZIERTER ZENTRALER APNOE

SYSTÈME POUR ESTIMER LA RÉSISTANCE DES VOIES AÉRIENNES SUPÉRIEURES ET LA COMPLIANCE PULMONAIRE AU MOYEN D'APNÉES CENTRALES INDUITES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2010 US 345221 P**

(43) Date of publication of application:
**27.03.2013 Bulletin 2013/13**

(73) Proprietor: **Koninklijke Philips N.V.**
**5656 AE Eindhoven (NL)**

(72) Inventor: **O'CONNOR, Nathan, Francis**
**Briarcliff Manor, New York 10510-8001 (US)**

(74) Representative: **van Velzen, Maaike Mathilde**
**Philips Intellectual Property & Standards**
**High Tech Campus 5**
**5656 AE Eindhoven (NL)**

(56) References cited:
**EP-A1- 0 651 971**

• **FARRE R ET AL: "SERVOCONTROLLED GENERATOR TO MEASURE RESPIRATORY IMPEDANCE FROM 0.25 TO 26 HZ IN VENTILATED PATIENTS AT DIFFERENT PEEP LEVELS", EUROPEAN RESPIRATORY JOURNAL, MUNKSGAARD INTERNATIONAL PUBLISHERS, COPENHAGEN, DK, vol. 8, no. 7, 1 July 1995 (1995-07-01), pages 1222-1227, XP001203575, ISSN: 0903-1936, DOI: 10.1183/09031936.95.08071222**
• **MOHY G. MORRIS: "a novel physiological investigation of the functional residual capacity by the bias flow nitrogen washout technique in infants", PEDIATRIC PULMONOLOGY, no. 44, 4 June 2009 (2009-06-04), pages 683-692, XP008140852,**

## Description

The present invention relates to a system for estimating the upper airway resistance and/or lung compliance of a subject, and, in particular, a system for estimating the airway resistance and/or lung compliance of a subject wherein a central apnea is induced in the subject to enable the estimation to be made simply and effectively without having to account for pressure generated by respiratory muscles (Pmus).

Pressure support ventilation systems that provide a flow of breathing gas to an airway of a patient at an elevated pressure to augment or substitute the patient's own ventilatory effort are well known. For example, a mechanical ventilation technique known as pressure support ventilation (PSV) is commonly used to decrease work of breathing in patients that require ventilatory assistance. During PSV, the ventilator applies constant pressure each time the patient's inspiratory effort is detected. Proportional assist ventilation (PAV) is another type of mechanical ventilation technique that provides dynamic inspiratory pressure assistance in linear proportion to patient-generated volume and flow. Another ventilation technique that is often used to treat patients with acute lung injury is known as airway pressure release ventilation (APRV). In APRV mode, a flow of breathing gas, such as air, is provided to a patient's airway at two different, alternating positive pressure levels (often referred to as PEEP High and PEEP Low) to provide ventilation and lung inflation while allowing the patient to spontaneously breath at both pressure levels. In addition, it is also well known to use a pressure support system to deliver positive airway pressure (continuous (CPAP) or variable) to treat a medical disorder such as sleep apnea syndrome or congestive heart failure.

In providing ventilatory assistance to patients, such as in the various ventilation therapies described above, it is often helpful and/or necessary to be able to obtain an estimate of the upper airway resistance and/or the lung compliance of the patient. However, estimating upper airway resistance and/or lung compliance in mechanically ventilated patients who have spontaneous respiratory efforts is rather complex, primarily due to that fact that knowledge of the force applied to the respiratory system is required and the fact that, in ventilated patients who have spontaneous respiratory efforts, that force includes a component related to pressure generated by respiratory muscles (Pmus), which continuously changes during the inflation phase of ventilation.

Additionally, quantification of lung compliance may be a useful tool in evaluating the health of a subject, including detection of fluid retention associated with developing acute congestive heart failure.

In Farré et al., "Servocontrolled generator to measure respiratory impedance from 0.25 to 26 Hz in ventilated patients at different PEEP levels", The European Respiratory Journal, 1995, 8(7): 1222-1227, the authors describe the design of a forced oscillation generator operating from spontaneous breathing frequencies whilst withstanding PEEP. This generator is based on a servocontrolled loudspeaker and permits the assessment of respiratory mechanics over a wide frequency band ranging from breathing frequencies to the most typical forced oscillation frequencies during end-expiratory pauses at PEEPs within the conventional range.

In Morris, "A Novel Physiological Investigation of the Functional Residual Capacity by the Bias Flow Nitrogen Washout Technique in Infants", Journal of Pediatric Pulmonology, Volume 44, pages 683-692, 2009, the author describes a technique to measure the passive static functional residual capacity by inducing a brief post-hyperventilation apnea in 33 healthy infants. In the study a commercial system for nitrogen washout to measure functional residual capacity and a custom made system to monitor and record flow and airway opening pressure signals in real-time are used in unison. Infants are manually hyperventilated to induce a post-hyperventilation apnea. After the last passive expiration the functional residual capacity is estimated by modifying the breathing gas composition during the post-expiratory apneic pause and making use of the washout technique.

Thus, there is a need for a system for simply and effectively estimating the airway resistance and/or lung compliance of a subject, including ventilated patients who have spontaneous respiratory efforts.

A method of estimating upper airway resistance or lung compliance of a patient is provided that includes inducing a central apnea in the patient, wherein inducing the central apnea comprises providing a central apnea inducing stimulus to the patient and determining whether patient airflow has ceased. The method further comprises: providing a known pressure stimulus comprising a flow of breathing gas having a known pressure level to the patient while the patient is experiencing the central apnea, determining a patient flow-related parameter, such as flow rate and/or flow volume, associated with the known pressure stimulus, and estimating the upper airway resistance or the lung compliance of the patient using the patient flow-related parameter using, for example and without limitation, an input-output system identification method. Providing the known pressure stimulus, determining the patient flow-related parameter and estimating the upper airway resistance or the lung compliance are performed only if it is determined that patient airflow has ceased.

A system according to claim 1 including a pressure generating system, a patient circuit and a controller is provided that implements that method just described.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

Figure 1 is a schematic diagram of an exemplary positive airway pressure support system suitable for estimating airway resistance and/or lung compliance according to the principles of the present invention;

FIG. 2 is a flowchart showing a method of estimating the airway resistance and/or lung compliance of a subject wherein a central apnea is induced in the subject according to one particular, non-limiting embodiment of the invention;

FIG. 3 is a circuit diagram used in a method for estimating upper airway resistance and/or lung compliance employing an input-output system identification method utilizing a single-compartment lung model according to one exemplary embodiment.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As employed, herein, the statement that two or more parts or components are "coupled" together shall mean that the parts are joined or operate together either directly or through one or more intermediate parts or components.

As employed herein, the statement that two or more parts or components "engage" one another shall mean that the parts exert a force against one another either directly or through one or more intermediate parts or components.

As employed herein, the term "number" shall mean one or an integer greater than one (i.e., a plurality).

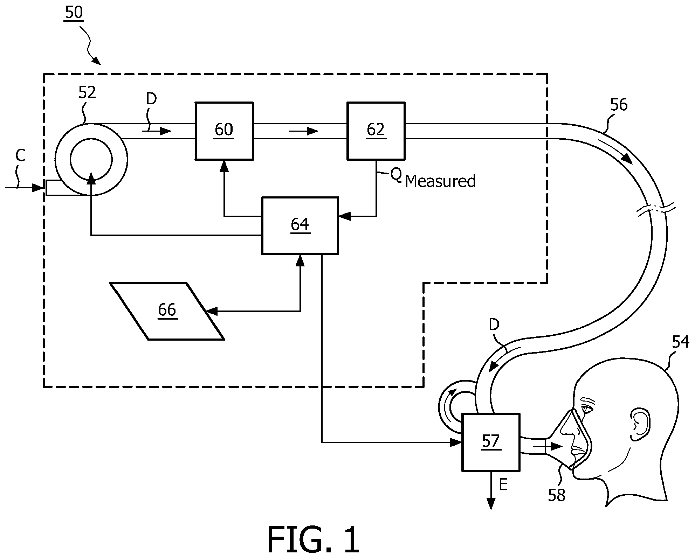

FIG. 1 schematically illustrates an exemplary positive airway pressure support system 50 suitable for estimating upper airway resistance and/or lung compliance according to the principles of the present invention. Pressure support system 50 includes gas flow/pressure generator 52, such as a blower used in a conventional CPAP or bi-level pressure support device, piston, bellows, compressor, or any other device that receives breathing gas, generally indicated by arrow C, from any suitable source, e.g., a pressurized tank of oxygen or air, the ambient atmosphere, or a combination thereof. Gas flow/pressure generator 52 generates a flow of breathing gas, such as air, oxygen, or a mixture thereof, for delivery to an airway of a patient 54 at relatively higher and lower pressures, i.e., generally equal to or above ambient atmospheric pressure.

The pressurized flow of breathing gas, generally indicated by arrow D from gas flow/pressure generator 52 is delivered, via a delivery conduit 56, to breathing mask or patient interface 58 of any known construction, which is typically worn by or otherwise attached to patient 54 to communicate the flow of breathing gas to the airway of the patient. Delivery conduit 56 and patient interface device 58 are typically collectively referred to as a patient circuit.

Although not shown in FIG. 1, the present invention also contemplates providing a secondary flow of gas, either alone or in combination with the primary flow of gas (arrow C) from atmosphere. For example, a flow of oxygen from any suitable source, such as an oxygen concentrator, or oxygen storage device (liquid or gas), can be provided upstream of gas flow/pressure generator 52 or downstream of the gas flow generator, for example, in the patient circuit or at the patient interface device, to control the fraction of inspired oxygen delivered to the patient.

Pressure support system 50 shown in FIG. 1 is a single-limb system, meaning that the patient circuit includes only delivery conduit 56 connecting the patient to the pressure support device. As such, active exhaust valve 57 (controlled by controller 64 described below) is provided in the delivery conduit 56 for, at times, venting exhaled gasses from the system to atmosphere as indicated by arrow E. In addition, exhaust valve 57 also be selectively actuated (under the control of controller 64) to allow exhaled gasses to re-enter delivery conduit 56 and be re-breathed by patient 54. The purpose of this functionality in the present invention is described elsewhere herein. It should be noted that the exhaust valve 57 can be provided at other locations in addition to or instead of in the delivery conduit, such as in the patient interface device 58. It should also be understood that exhaust valve 57 can have a wide variety of configurations depending on the desired manner in which gas is to be vented from the pressure support system.

The present invention also contemplates that the variable positive airway pressure support system can be a two-limb system, having a delivery conduit and an exhaust conduit connected to the patient. In a two-limb system, the exhaust conduit carries exhaust gas from the patient and includes an exhaust valve at the end distal from the patient. The exhaust valve is typically actively controlled to maintain a desired level of pressure in the system, which is commonly known as positive end expiratory pressure (PEEP). This is accomplished by controlling the flow of exhaust gas from the otherwise closed system.

In the illustrated exemplary embodiment of the present invention, patient interface 58 is a nasal/oral mask. It is to be understood, however, that patient interface 58 can include a nasal mask, nasal pillows, tracheal tube, endotracheal tube, or any other device that provides the gas flow communicating function. Also, for purposes of the present invention, the phrase "patient interface" can include delivery conduit 56 and any other structures that connect the source of pressurized breathing gas to the patient.

It is to be understood that various components may be provided in or coupled to the patient circuit. For example, a bacteria filter, pressure control valve, flow control valve, sensor, meter, pressure filter, humidifier and/or heater can be provided in or attached to the patient circuit. Likewise, other components, such as muffler and filters can be provided at the inlet of gas flow/pressure generator 52 and at the outlet of valve 60 (described below).

In the illustrated embodiment, variable positive airway pressure support system 50 includes a pressure controller in the form of a valve 60 provided in delivery conduit 56. Valve 60 controls the pressure of the flow of breathing gas from gas flow/pressure generator 52 delivered to patient 54. For present purposes, gas flow/pressure generator 52 and valve 60 are collectively referred to as a "pressure generating system" because they act in concert to control the pressure and/or flow of gas delivered to the patient.

It should be apparent that other techniques for controlling the pressure delivered to the patient by the gas flow/pressure generator, such as varying the blower speed, either alone or in combination with a pressure control valve, are contemplated by the present invention. Thus, valve 60 is optional depending on the technique used to control the pressure of the flow of breathing gas delivered to the patient. If valve 60 is eliminated, the pressure generating system corresponds to gas flow/pressure generator 52 alone, and the pressure of gas in the patient circuit is controlled, for example, by controlling the motor speed of the gas flow/pressure generator.

Pressure support system 50 further includes flow sensor 62 that measures the flow of breathing gas within delivery conduit 56. In accordance with the exemplary embodiment shown in FIG. 1, flow sensor 62 is interposed in line with delivery conduit 56, most preferably downstream of valve 60. Flow sensor 62 generates a flow signal $Q_{measured}$ that is provided to controller 64 and is used by controller 64 to determine the flow of gas at the patient $Q_{patient}$.

Techniques for calculating $Q_{patient}$ based on $Q_{measured}$ are well known, and take into consideration the pressure drop of the patient circuit, known leaks from the system, i.e., the intentional exhausting of gas from the circuit as indicated by arrow E in FIG. 1, and unknown leaks from the system, such a leaks at the mask/patient interface. The present invention contemplates using any conventional technique for calculating leak flow $Q_{leak}$, and using this determination in calculating $Q_{patient}$ based on $Q_{measured}$. Examples of such techniques are taught by U.S. Patent Nos. 5,148,802; 5,313,937; 5,433,193; 5,632,269; 5,803,065; 6,029,664; 6,539,940; 6,626,175; and 7,011,091, and by U.S. patent application publication no. 2003/0066528, the contents of each of which are incorporated by reference into the present invention.

Other techniques for measuring the patient flow of patient 54 are contemplated by the present invention. For example, the flow can be measured directly at patient 54, in which case the measured flow corresponds directly the patient flow $Q_{patient}$ and no flow estimation is necessary. The present invention also contemplates measuring the flow at other locations along delivery conduit 56.

In addition, the present invention contemplates determining the estimated patient flow $Q_{patient}$ based on other characteristics of the pressure support system. For example, the operation of the gas flow/pressure generator or a flow/pressure controller, such as a valve, is affected by the flow in the patient circuit, or by the systems attempt to maintain the pressure in the system. As a result, monitoring a characteristic of the system, such as monitoring the power, torque, and/or rotating speed of the pressure generator or the position of the valve, can be used as a surrogate for measuring the patient flow directly. It is also known to measure patient flow using a flow sensor upstream of the gas flow/pressure generator. Of course, any combination of such flow measuring techniques can also be used. In these latter cases, an estimation of patient flow $Q_{patient}$ based on the measured flow or other parameter will be needed.

Furthermore, as is well known in the art, controller 64 may determine the volume of the gas delivered to patient 54 by integrating patient flow data (based on data provided by flow sensor 62 and determined in any of the manners described herein or any other suitable manner).

Controller 64 includes a processing portion which may be, for example, a microprocessor, a microcontroller or some other suitable processing device, and a memory portion that may internal to the processing portion or operatively coupled to the processing portion and that provides a storage medium for data and software executable by the processing portion for controlling the operation of pressure support system 50, including estimating the airway resistance and/or lung compliance of patient 54 as described in greater detail herein.

Input/output device 66 is provided for setting various parameters used by the variable positive airway pressure support system, as well as for displaying and outputting information and data to a user, such as a clinician or caregiver. It is to be understood that the present invention contemplates providing input/output terminals so that the operation information and data collected by the pressure support system can be monitored and controlled remotely.

As described elsewhere herein, estimating upper airway resistance and/or lung compliance in a subject requires knowledge of the force applied to the respiratory system of the subject. In mechanically ventilated patients who have spontaneous respiratory efforts, that force includes two components: (i) a known component in the form of the pressure applied to the respiratory system by a pressure signal (i.e., a delivered breathing gas) from a pressure support system such as pressure support system 50, and (ii) an unknown component resulting from the diaphragmatic pressure ($P_{mus}$). Estimating upper airway resistance and/or lung compliance for such patient is made particularly complex because typically it is necessary to compensate/account for this unknown component and because this unknown component varies with each respiratory cycle. However, if a period exists where the patient applies no diaphragmatic effort, there would be no need to compensate/account for the unknown component in the upper airway resistance and/or lung compliance estimation, and, as a result, an input-output system identification approach or method using a model of the human lungs can be directly applied to measured patient data to estimate upper airway resistance and/or lung compliance.

One period where the patient applies no diaphragmatic effort is during a central apnea. In particular, a central apnea occurs when there is a cessation of airflow as a result of a lack of respiratory drive. The present invention provides a method of estimating upper airway resistance and/or lung compliance wherein a central apnea is induced in the patient in order to create a period where the unknown component described above is eliminated, and to therefore allow the upper airway resistance and/or lung compliance to be more simply estimated using an input-output system identification approach that employs a model of the human lungs, such as, without limitation, a known single compartment lung model. An example of a single compartment lung model is described in United States Patent Application Publication No. 2004/0097821, entitled "Method and Breathing Apparatus for Assessing Pulmonary Stress," the disclosure of which is incorporated herein by reference.

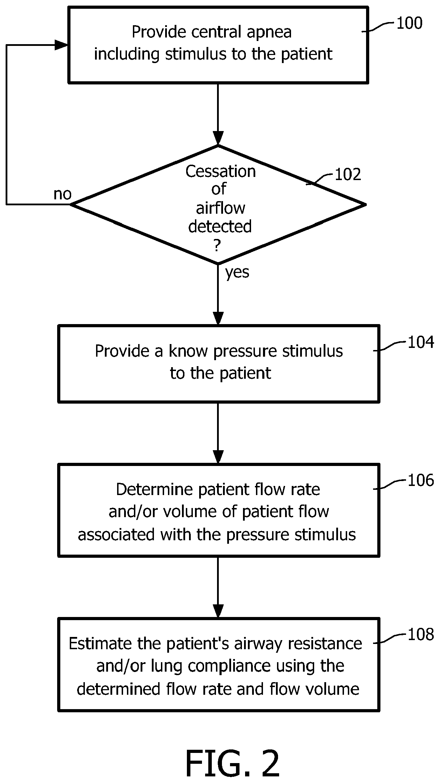

FIG. 2 is a flowchart showing a method of estimating the upper airway resistance and/or lung compliance of a subject wherein a central apnea is induced in the subject to enable the estimation to be made simply and effectively according to one particular, non-limiting embodiment of the invention. The method shown in FIG. 2 may be implemented in the exemplary pressure support system 50 shown in FIG. 1 (or in another suitable pressure support system) through appropriate programming of controller 64. For illustrative purposes, the method will be described herein as implemented in the pressure support system 50. In addition, the method shown in FIG. need not be repeated continuously. Instead, it may be performed on a periodic basis. Additionally, the frequency with which the method is performed may depend on the quality of the estimation of resistance and/or compliance that is obtained (the higher the quality, the less frequently it is performed). In the exemplary embodiment described below, least-squared error estimation is employed, and the squared error is commonly used as an assessment of the "quality of fit" and therefore indicates the quality of the estimation

The method of FIG. 2 begins at step 100, wherein a central apnea inducing stimulus is provided to the patient using pressure support system 50. The central apnea inducing stimulus may take a number of different forms. For example, pressure support system 50 may increase the tidal volume of the patient for a given respiratory effort to a point in which the patient's $PCO_2$ level falls below the apneic threshold. This may be accomplished by causing pressure support system 50 to provide bi-level pressure with a high level of pressure support (i.e., the difference between the inspiratory pressure and the expiratory pressure) until a central apnea occurs. Alternatively, active valve 57 could be actuated in a manner that allows exhaled air, and thus $CO_2$, to reenter the patient circuit, thereby causing $CO_2$ rebreathing to occur. This would cause hyperventilation and a drop in $PCO_2$ below the apneic threshold to induce a central apnea. These methods, and/or other suitable central apnea inducing methods, could be applied independently or in combination to induce a central apnea.

Next, at step 102, a determination is made as to whether a cessation of airflow has been detected based on the output of flow sensor 62. The cessation of airflow would indicate a central apnea has been successfully induced in the patient. If the answer at step 102 is no, then the method returns to step 100, where efforts to induce a central apnea are repeated. If, however, the answer at step 102 is yes, meaning a central apnea has been successfully induced in the patient, then the method proceeds to step 104. At step 104, a known pressure stimulus in the form of a flow of breathing gas at a known pressure level is provided to patient 54 by pressure support system 50 through delivery conduit 56 and patient interface 58. For example, and without limitation, the pressure stimulus could be a step change in the pressure level of the flow of breathing gas provided by pressure support system 50. Next, at step 106, the flow rate of gas at the patient 54 ($Q_{patient}$) and/or the volume of the flow of gas at the patient 54 associated with the provided known pressure stimulus is determined. The flow rate of gas at the patient 54 ($Q_{patient}$) may be determined by controller 64 in any of the manners described herein (e.g., based on the output of flow sensor 62) or in any other suitable manner. In addition, as described elsewhere herein, the volume of the flow of gas at the patient 54 may be determined by controller 64 by integrating the patient flow rate or in any other suitable manner. As will be appreciated, steps 104 and 106 are performed during a period where patient 54 is experiencing a central apnea and thus when patient 54 is exerting no diaphragmatic effort.

Following step 106, the method proceeds to step 108, wherein the upper airway resistance and/or lung compliance of patient 54 is/are estimated using either or both of the determined patient flow rate and patient flow volume. In the exemplary embodiment, upper airway resistance and/or lung compliance is/are determined directly from either or both of the determined patient flow rate and patient flow volume using an input-output system identification method based on, for example, a single compartment lung model.

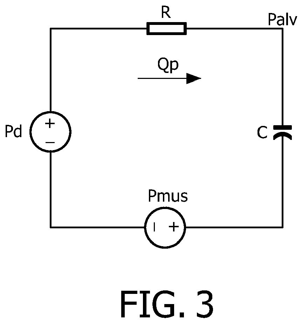

In one particular, non-limiting embodiment, upper airway resistance and/or lung compliance is/are determined directly using an input-output system identification method utilizing a single-compartment lung model in the following manner. The transfer function in the s-domain relating patient flow to pressure of the device and the diaphragm of the subject for the circuit in FIG. 3 is given by:

$$(1) \quad \frac{Q_p(s)}{P(s)} = \frac{Cs}{RCs + 1},$$

where

$$(2) \quad P(s) = P_d(s) + P_{mus}(s) = P_d(s).$$

Additionally, the patient volume is given by the equation

$$(3) \quad V(s) = \frac{Q_p(s)}{s}.$$

Thus, the transfer function relating the pressure to patient volume is given by the equation:

$$(4) \quad \frac{V(s)}{P(s)} = \frac{C}{RCs + 1} \Rightarrow V(s) = \frac{C}{RCs + 1} P_d(s);$$

Pd(s) represents the pressure of the pressurized flow of breathable gas generated by a pressure support device. Since the pressures and volumes, for the inhalation are known, any one of various known numerical estimation techniques can be used to determine resistance R and compliance C. By way of non-limiting example, the technique of least-squared error could be implemented.

Moreover, one or more parameters relating to the flow of breathing gas at the patient other than patient flow rate and patient flow volume may be determined in step 106 and then used to directly to estimate upper airway resistance and/or lung compliance.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

**Claims**

1. A system (50) for estimating upper airway resistance or lung compliance, comprising:

   a pressure generating system (52);
   a patient circuit (56) operatively coupled to the pressure generating system; and
   a controller (64) operatively coupled to the pressure generating system, the controller being adapted to estimate the upper airway resistance or the lung compliance of a patient by:

   inducing a central apnea in the patient, wherein inducing the central apnea comprises causing the pressure generating system to provide a central apnea inducing stimulus to the patient and determining whether patient airflow has ceased;
   causing the pressure generating system to generate a known pressure stimulus comprising a flow of breathing gas having a known pressure level while the patient is experiencing the central apnea, wherein the known pressure stimulus is provided to the patient through the patient circuit;
   determining a patient flow-related parameter associated with the known pressure stimulus; and
   estimating the upper airway resistance or the lung compliance of the patient using the patient flow-related parameter;
   wherein causing the pressure generating system to generate a known pressure stimulus, determining the patient flow-related parameter and estimating the upper airway resistance or the lung compliance are performed only if it is determined that patient airflow has ceased

2. The system according to claim 1, wherein the patient flow-related parameter is a system according to claim 8, wherein the patient flow-related parameter is a volume of flow at the patient.

3. The system according to claim 1, wherein the estimating employs an input-output system identification method.

4. The system according to claim 3, wherein the input-output system identification method is based on a single compartment lung model.

5. The system according to claim 3, wherein the causing the pressure generating system to provide a central apnea inducing stimulus to the patient comprises causing the pressure generating system to increase a tidal volume of gas delivered to the patient to a point that causes the patient's $PCO_2$ level to fall below an apneic threshold.

6. The system according to claim 3, wherein the causing the pressure generating system to provide a central apnea inducing stimulus to the patient comprises causing the patient circuit to allow $CO_2$ rebreathing to occur in the patient to cause the patient's $PCO_2$ level to fall below an apneic threshold.

**Patentansprüche**

1. System (50) zum Einschätzen der Resistenz der oberen Atemwege oder der Lungencompliance, umfassend:

ein Druckgenerierungssystem (52);
ein Einschlauchsystem (56), das betriebsfähig mit dem Druckgenerierungssystem gekoppelt ist; und
einen Controller (64), der betriebsfähig mit dem Druckgenerierungssystem gekoppelt ist, wobei der Controller angepasst ist, um die Resistenz der oberen Atemwege oder die Lungencompliance eines Patienten einzuschätzen durch:

Induzieren einer zentralen Apnoe bei dem Patienten, wobei das Induzieren der zentralen Apnoe das Bewirken, dass das Druckgenerierungssystem einen eine zentrale Apnoe induzierenden Reiz für den Patienten bereitstellt, und das Bestimmen, ob der Atemfluss des Patienten aufgehört hat, umfasst;
Bewirken, dass das Druckgenerierungssystem einen bekannten Druckreiz generiert, der einen Atemgasstrom umfasst, der einen bekannten Druckpegel aufweist, während der Patient die zentrale Apnoe erfährt, wobei der bekannte Druckreiz dem Patienten über das Einschlauchsystem bereitgestellt wird;
Bestimmen eines Parameters bezüglich des Patientenflusses, der mit dem bekannten Druckreiz verknüpft ist; und
Einschätzen der Resistenz der oberen Atemwege oder der Lungencompliance des Patienten unter Verwendung des Parameters bezüglich des Patientenflusses;
wobei das Bewirken, dass das Druckgenerierungssystem einen bekannten Druckreiz generiert, das Bestimmen des Parameters bezüglich des Patientenflusses und das Einschätzen der Resistenz der oberen Atemwege oder der Lungencompliance nur ausgeführt werden, falls bestimmt wird, dass der Atemfluss des Patienten aufgehört hat.

2. System nach Anspruch 1, wobei der Parameter bezüglich des Patientenflusses ein System nach Anspruch 8 ist, wobei der Parameter bezüglich des Patientenflusses ein Durchflussvolumen am Patienten ist.

3. System nach Anspruch 1, wobei das Einschätzen ein Verfahren zum Identifizieren eines Ein-/Ausgabesystems verwendet.

4. System nach Anspruch 3, wobei das Verfahren zum Identifizieren eines Ein-/Ausgabesystems auf einem Einkammer-Lungenmodell basiert.

5. System nach Anspruch 3, wobei das Bewirken, dass das Druckgenerierungssystem einen eine zentrale Apnoe induzierenden Reiz für den Patienten bereitstellt, das Bewirken umfasst, dass das Druckgenerierungssystem ein Tidalvolumen von Gas, das an den Patienten abgegeben wird, so weit erhöht, dass bewirkt wird, dass der $PCO_2$-Pegel des Patienten unter eine apnoische Schwelle abfällt.

6. System nach Anspruch 3, wobei das Bewirken, dass das Druckgenerierungssystem einen eine zentrale Apnoe induzierenden Reiz für den Patienten bereitstellt, das Bewirken umfasst, dass das Einschlauchsystem zulässt, dass eine $CO_2$-Rückatmung bei dem Patienten vorkommt, um zu bewirken, dass der $PCO_2$-Pegel des Patienten unter eine apnoische Schwelle abfällt.

**Revendications**

1. Système (50) pour estimer la résistance des voies aériennes supérieures ou la compliance pulmonaire, comprenant :

un système de génération de pression (52) ;
un circuit patient (56) opérationnellement raccordé au système de génération de pression ; et
un système de commande (64) opérationnellement raccordé au système de génération de pression, le système de commande étant adapté pour estimer la résistance des voies aériennes supérieures ou la compliance pulmonaire d'un patient par :

l'induction d'une apnée centrale chez le patient, dans lequel l'induction de l'apnée centrale comprend le déclenchement par le système de génération de pression d'un stimulus induisant une apnée centrale pour le patient et déterminant si le débit d'air du patient a cessé ;

la génération par le système de génération de pression d'un stimulus de pression connu comprenant un débit de gaz respiratoire ayant un niveau de pression connu tandis que le patient expérimente l'apnée centrale, dans lequel le stimulus de pression connu est fourni au patient à travers le circuit patient ;

la détermination d'un paramètre relatif au débit du patient associé au stimulus de pression connu ; et

l'estimation de la résistance des voies aériennes supérieures ou de la compliance pulmonaire du patient en utilisant le paramètre relatif au débit du patient ;

dans lequel la génération par le système de génération de pression d'un stimulus de pression connu, la détermination du paramètre relatif au débit du patient et l'estimation de la résistance des voies aériennes supérieures ou de la compliance pulmonaire sont réalisées uniquement s'il est déterminé que le débit d'air du patient a cessé.

2.  Système selon la revendication 1, dans lequel le paramètre associé au débit du patient est un système selon la revendication 8, dans lequel le paramètre associé au débit du patient est un volume de flux au patient.

3.  Système selon la revendication 1, dans lequel l'estimation utilise un procédé d'identification du système d'entrée-sortie.

4.  Système selon la revendication 3, dans lequel le procédé d'identification du système d'entrée-sortie est basé sur un modèle pulmonaire à compartiment unique.

5.  Système selon la revendication 3, dans lequel la génération de pression par le système pour fournir un stimulus induisant une apnée centrale au patient comprend l'augmentation par le système de génération de pression d'un volume courant de gaz fourni au patient en un point qui provoque la chute du niveau de $PCO_2$ du patient en-deçà d'un seuil apnéique.

6.  Système selon la revendication 3, dans lequel la fourniture par le système de génération de pression d'un stimulus induisant une apnée centrale au patient comprend le fait de permettre au circuit patient de respirer à nouveau du $CO_2$, pour faire en sorte que le niveau de $PCO_2$ du patient chute en deçà d'un seuil apnéique.

FIG. 1

Provide central apnea
including stimulus to the patient ⌐100

Cessation
of
airflow
detected
? ⌐102

no

yes

Provide a know pressure stimulus
to the patient ⌐104

Determine patient flow rate
and/or volume of patient flow
associated with the pressure stimulus ⌐106

Estimate the patient's airway resistance
and/or lung compliance using the
determined flow rate and flow volume ⌐108

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5148802 A
- US 5313937 A
- US 5433193 A
- US 5632269 A
- US 5803065 A
- US 6029664 A
- US 6539940 B
- US 6626175 B
- US 7011091 B
- US 20030066528 A
- US 20040097821 A

**Non-patent literature cited in the description**

- **FARRÉ et al.** Servocontrolled generator to measure respiratory impedance from 0.25 to 26 Hz in ventilated patients at different PEEP levels. *The European Respiratory Journal,* 1995, vol. 8 (7), 1222-1227
- **MORRIS.** A Novel Physiological Investigation of the Functional Residual Capacity by the Bias Flow Nitrogen Washout Technique in Infants. *Journal of Pediatric Pulmonology,* 2009, vol. 44, 683-692